# EUROPÄISCHE PATENTSCHRIFT

(11) **EP 1 888 129 B1**
(45) Veröffentlichungstag und Bekanntmachung des Hinweises auf die Patenterteilung: **16.09.2009**
(21) Anmeldenummer: 06755233.1
(22) Anmeldetag: 17.05.2006
(51) Int. Cl.: A61L 9/014, B01J 20/26, A61L 101/42

(54) **SUSPENSION ZUR VERMINDERUNG VON GERUCH**
SUSPENSION FOR REDUCING ODOURS
SUSPENSION POUR EVITER LES ODEURS

(30) Priorität: 24.05.2005 DE 102005023857
(43) Veröffentlichungstag der Anmeldung: 20.02.2008
(73) Patentinhaber: BASF SE, 67056 Ludwigshafen (DE)
(72) Erfinder: MÜLLER, Ulrich, 67435 Neustadt (DE); HESSE, Michael, 67549 Worms (DE); PÜTTER, Hermann, 67433 Neustadt (DE); SCHUBERT, Markus, 67063 Ludwigshafen (DE); GUZMANN, Marcus, 69242 Mühlhausen (DE); HUFF, Jürgen, 67063 Ludwigshafen (DE)
(74) Vertreter: Isenbruck, Günter
(86) Internationale Anmeldenummer: PCT/EP2006/062376
(87) Internationale Veröffentlichungsnummer: WO 2006/125739

(56) Entgegenhaltungen:
- EP-A- 1 574 158
- WO-A-00/23119
- WO-A-03/102000
- CHAE HEE K ; SIBERIO-PEREZ DIANA Y ; KIM JAHEON ; GO YONGBOK ; EDDAOUDI MOHAMED ; MATZGER ADAM J ; O'KEEFFE MICHAEL ; YAGHI OMAR M: "A route to high surface area, porosity and inclusion of large molecules in crystals" NATURE, Bd. 427, 5. Februar 2004 (2004-02-05), Seiten 523-527, XP002386278
- O'KEEFFE M; EDDAOUDI M; LI H; REINEKE T; YAGHI O M: "Frameworks for extended solids: geometrical design principles" JOURNAL OF SOLID STATE CHEMISTRY, Bd. 152, 1. Juni 2000 (2000-06-01), Seiten 3-20, XP002386279 in der Anmeldung erwähnt

## Beschreibung

Die vorliegende Erfindung betrifft eine Suspension sowie Verfahren und Verwendung einer solchen Suspension zur Verminderung von Geruch.

Gerüche, insbesondere sogenannte schlechte Gerüche, stellen insbesondere im täglichen Leben ein Problem dar.

Insbesondere bei sogenannten schlechten Gerüchen wird der Mensch durch Wahrnehmung über dessen Geruchssinn in seiner Empfindung negativ beeinflusst.

Dies kann häufig dadurch vermindert oder umgekehrt werden, in dem geruchsbildende Stoffe eingesetzt werden, die vom Menschen allgemein als gut riechend empfunden werden. Dadurch soll der schlechte Geruch zurückgedrängt werden.

Häufig wird der vom Menschen wahrgenommene Geruch durch Geruchsstoffe verursacht, die unabhängig vom subjektiven Geruchsempfinden schädlich für den menschlichen Organismus sind. Hierbei kann der Einsatz "gut riechender" Substanzen das problematische Vorhandensein gesundheitsbeeinträchtigender Stoffe nicht vermindern.

Eine weitere Möglichkeit besteht darin, den durch Geruchsstoffe erzeugten Geruch dadurch zu beseitigen, dass die Geruchsstoffe chemisch zersetzt werden, beispielsweise durch Enzyme.

Eine alternative Möglichkeit besteht darin, Gerüche dadurch zu vermindern, dass die die Gerüche verursachenden Geruchsstoffe an bestimmte Materialien sorbiert werden. Diese Sorbentien können - meist verdünnt - durch Zerstäuber an den gewünschten Wirkungsort gebracht werden.

Ein solches im Stand der Technik bekanntes Sorbens stellt Cyclodextrin dar.

Ein Zerstäuber enthaltend eine Suspension von Molekularsieben zur Verminderung von Geruch ist aus der WO 00/23119 bekannt.

Nachteilig an den im Stand der Technik bekannten Sorbentien oder Lösungen beziehungsweise Suspensionen oder Emulsionen, in denen die Sorbentien vorliegen, ist deren teilweise geringe Effizienz zur Verminderung des unerwünschten Geruchs.

Es besteht daher ein Bedarf, Alternativen zu den im Stand der Technik bekannten Lösungen oder Suspensionen, insbesondere für die Zerstäubung bereitzustellen.

Die Aufgabe der vorliegenden Erfindung besteht somit darin, alternative Suspensionen sowie Verfahren zur Verminderung von Geruch bereitzustellen, wobei das in der Suspension vorliegende Sorbens verbesserte Eigenschaften gegenüber solchen des Standes der Technik aufweisen soll.

Die Aufgabe wird gelöst durch eine Suspension zur Verminderung von Geruch gemäss Anspruch 1 enthaltend ein poröses metallorganisches Gerüstmaterial in einer Flüssigkeit, wobei das Gerüstmaterial mindestens eine an mindestens ein Metallion koordinativ gebundene, mindestens zweizähnige organische Verbindung enthält.

Die Aufgabe wird ebenfalls gelöst durch ein Verfahren zur Vermindung von Geruch, den Schritt enthaltend
- Inkontaktbringen eines den Geruch enthaltenden Gases oder an der Oberfläche eines Gegenstandes oder an einem Lebewesen haftenden Geruches mit einer erfindungsgemäßen Suspension.

Es wurde nämlich gefunden, dass durch die Verwendung eines wie oben beschriebenen porösen metallorganischen Gerüstmaterials eine effiziente Verminderung von Geruch stattfinden kann.

Der Singular sowie der Plural des Begriffs "Geruch" werden im Rahmen der vorliegenden Erfindung synonym verwendet. Hierbei stellt der Begriff Geruch jede über den Geruchssinn des Menschen potentiell wahrnehmbare Empfindung dar, die durch einen oder mehrere Geruchsstoffe erzeugt werden kann.

In diesem Zusammenhang bedeutet der Begriff "potentiell", dass der Geruchsstoff oder die Geruchsstoffe, die einen Geruch erzeugen, im Rahmen der vorliegenden Erfindung nicht in einer Konzentration vorliegen müssen, die eine Wahrnehmung über den menschlichen Geruchssinn ermöglichen. Erfindungsgemäß reicht somit die bloße Anwesenheit eines solchen Geruchsstoffes oder solcher Geruchsstoffe aus.

Vorzugsweise liegen jedoch der Geruchsstoff oder die Geruchsstoffe in einer für den durchschnittlichen menschlichen Geruchssinn wahrnehmbaren Konzentration vor.

Die Konzentration des Gerüstmaterials in der Suspension liegt vorzugsweise im Bereich von 0,001 bis 5 Gew.-% bezogen auf das Gesamtgewicht der Suspension. Weiterhin bevorzugt liegt die Konzentration im Bereich von 0,01 bis 2,5 Gew.%, mehr bevorzugt im Bereich von 0,1 bis 1 Gew.-%, jeweils bezogen auf das Gesamtgewicht der Suspension vor.

Geeignete Flüssigkeiten zur Herstellung der Suspension sind solche, die Wasser enthalten. Insbesondere bevorzugt ist Wasser als Flüssigkeit.

Weiterhin bevorzugt sind Flüssigkeiten, die ein leichtes Zerstäuben der mit dem Gerüstmaterial gebildeten Suspension ermöglichen.

Durch die Zerstäubung kann eine besonders bevorzugte und effiziente Verteilung der Suspension, insbesondere durch feine Tropfenbildung, erreicht werden.

Demzufolge ist ein weiterer Gegenstand der vorliegenden Erfindung ein Zerstäuber enthaltend eine erfindungsgemäße Suspension.

Hierbei können handelsübliche Zerstäuber verwendet werden.

Die erfindungsgemäße Suspension kann weitere chemische Stoffe enthalten. Hierbei sind zum Beispiel Geruchsstoffe zu nennen, die einen für den Menschen als angenehm empfundenen Geruch verursachen. Solche Stoffe werden häufig auch als Riech- oder Duftstoffe bezeichnet.

Wie bereits oben ausgeführt, betrifft die vorliegende Erfindung auch ein Verfahren zur Verminderung von Geruch. Vorteilhafterweise erfolgt die Verminderung so stark, dass der den Geruch erzeugende Geruchsstoff oder die Geruchsstoffe nicht mehr durch den durchschnittlichen Geruchssinn eines Menschen wahrgenommen werden, i.e. der Geruch entfernt wird.

Bei dem Geruch kann es sich um Gerüche des täglichen Lebens handeln oder auch um verschiedenste Geruchsstoffe, die bei verschiedensten Anwendungen auftreten können, beinhalten. Beispielhaft seien genannt Küchengeruch, Schweißgeruch, Inkontinenzgeruch, Lebensmittelgeruch, wie beispielsweise Alkohol- oder Fischgeruch, Toilettengeruch oder Geruch, der durch Tabakrauch, wie beispielsweise Zigarettenrauch, verursacht wird.

Dem Fachmann ist bekannt, dass die verschiedensten Gerüche im Rahmen der vorliegenden Erfindung vermindert werden können.

Der Geruch kann an einem Lebewesen haften. Bei dem Lebewesen kann es sich um einen Menschen oder ein Tier, wie beispielsweise einen Hund oder eine Katze, handeln. Das Inkontaktbringen des Lebewesens mit der Suspension kann dahingehend erfolgen, dass diese beziehungsweise die betroffenen Körperteile gleichmäßig mit der Suspension eingerieben werden oder beispielsweise mit Hilfe eines Zerstäubers die Suspension aufgebracht wird. Die erfindungsgemäße Suspension kann anschließend beispielsweise durch Waschen wieder entfernt werden.

Weiterhin kann der Geruch an der Oberfläche eines Gegenstandes haften. Hierbei sind die Begriffe "Oberfläche" sowie "Gegenstand" weitestgehend auszulegen. Im Rahmen der vorliegenden Erfindung liegt insbesondere immer dann ein Anhaften an einer Oberfläche eines Gegenstandes vor, wenn die den Geruch erzeugenden Geruchsstoffe oder der erzeugende Geruchsstoff mit der Suspension in Kontakt gebracht werden kann.

Gegenstände können verschiedenster Natur sein und beispielsweise aus dem täglichen Leben stammen. Hierbei sind beispielsweise zu nennen Gewebe und Stoffe, wie zum Beispiel Kleider, Polstermöbel, Gardinen oder Decken, Möbel aus Holz, Kunststoff oder einem anderen Material, Glasflächen wie Fenster, Tapeten, Wände und Decken, Fußböden, Teppiche oder ähnliches.

Die betroffenen Oberflächen können beispielsweise mit einem erfindungsgemäßen Zerstäuber mit Hilfe der erfindungsgemäßen Suspension befeuchtet werden. Zur Entfernung der Suspension kann diese weggewaschen oder weggewischt werden. Auch ein Verbleiben der Suspension an der Oberfläche kann unter gegebenen Umständen geeignet sein.

Weiterhin ist es möglich die Suspension zunächst auf einen nicht mit Geruch behafteten Suspensionsträger aufzubringen oder diesen mit der Suspension zu tränken und anschließend die mit Geruch behaftete Oberfläche eines Gegenstandes oder das Lebewesen mit Hilfe dieses Trägers mit der erfindungsgemäßen Suspension in Kontakt zu bringen. Ein solcher Träger kann beispielsweise ein herkömmliches Wischtuch oder dergleichen sein. Für den Fall eines mit Geruch behafteten Gases wie Luft kann ebenfalls ein solcher Träger zum Einsatz kommen. Hier eignen sich beispielsweise Filter, wie sie in vielfältiger Weise eingesetzt werden. So können Küchengerüche beispielsweise durch in Dunstabzugshauben enthaltenen Filter erfindungsgemäß vermindert werden.

Weiterhin kann die Geruchsverminderung ebenfalls ein Gas betreffen, in dem der Geruchsstoff oder die Geruchsstoffe, die den Geruch erzeugen, enthalten sind.

Im Rahmen der vorliegenden Erfindung wird vereinfachend der Begriff "Gas" auch dann verwendet, wenn es sich um Gasgemische wie beispielsweise Luft handelt. Bei den betreffenden Gasen ist es lediglich erforderlich, dass diese beim Inkontaktbringen mit der Suspension gasförmig vorliegen.

Vorzugsweise weist das Gas einen Siedepunkt oder -bereich auf, der unter der Raumtemperatur liegt. Es ist jedoch auch möglich, dass höhersiedende fluide Systeme zum Einsatz gelangen, wenn diese beispielsweise bei erhöhter Temperatur als Abgase freigesetzt werden und vor ihrer Kondensation mit der erfindungsgemäßen Suspension in Kontakt gelangen.

Vorzugsweise handelt es sich bei dem Gas um Erdgas, Biogas, Abgas, Luft, Abluft oder Inertgas. Mehr bevorzugt sind Erdgas, Biogas, Luft und Abluft. Insbesondere bevorzugt sind Biogas, Luft und Abluft. Ganz besonders bevorzugt ist Luft.

Das Gas kann in offenen oder zumindest teilweise geschlossenen Systemen vorliegen. Insbesondere im Fall von Erd- und Biogas kann es sich um Rohrleitungen, Pipelines, Kessel-, Transport- oder Erdgasbehälter, wie sie beispielsweise zur Lagerung in der Erde oder als Tanks für Kraftfahrzeuge verwendet werden, handeln. Im Falle von Abgasen handelt es sich vorzugsweise um Industrieabgase oder solche Abgase, wie sie bei Verbrennungsvorgängen (zum Beispiel bei Verbrennungsmotoren) anfallen. Weiterhin bevorzugt handelt es sich bei dem Gas um Raumluft in Gebäuden oder Räumen wie in Wohn- und Esszimmern oder insbesondere in Küchen. Auf die Raumluft in Fortbewegungsmitteln wie Personenkraftfahrzeuge, Lastkraftfahrzeuge, Züge oder Schiffe ist hierbei zu nennen. Ebenso ist die Raumluft in Geräten wie beispielsweise Geschirrspülmaschinen zu nennen.

Hierbei kann das Gas, das den Geruchsstoff oder die Geruchsstoffe, die den Geruch erzeugen, ebenfalls durch Zerstäuben der Suspension in Kontakt gebracht werden. Neben der Behandlung der Gase selbst kann zusätzlich auch die mit den Gasen in Kontakt befindlichen Systeme wie die Oberflächen der Innenwände der oben genannten Rohrleitungen, Pipelines, Kessel-, Transport- oder Erdgasbehälter mit der erfindungsgemäßen Suspension in Kontakt gebracht werden.

Insbesondere in den Fällen, bei denen das Gas Erdgas, Luft, Abluft oder Inertgas darstellt, kann der Geruchsstoff ursprünglich Bestandteil eines flüssigen (beispielsweise Wasser oder Erdöl) oder festen Mediums sein, der dann in die Phase des über der flüssigen oder festen Oberfläche anliegenden Gases übertritt und anschließend aus dieser entfernt werden. Beispielsweise kann es sich um ein Gas innerhalb einer Verpackung (Umgebungsgas) von festen Gegenständen handeln, die im Laufe der Zeit Geruchsstoffe innerhalb der Verpackung an das Umgebungsgas abgeben. Hierbei handelt es sich bei dem Umgebungsgas um Luft oder Inertgas. Ein weiteres Beispiel stellen Polymere dar, bei denen Monomere, die bei der Herstellung der Polymere nicht umgesetzt wurden, wobei diese jedoch noch im Polymer verblieben und im Laufe der Zeit an das Umgebungsgas, wie beispielsweise die Raumluft, abgegeben werden und die abzutrennenden Geruchsstoffe darstellen. Ebenso können im Polymer weitere leicht flüchtige Komponenten enthalten sein, die in das Umgebungsgas abgegeben werden können. Hierbei sind beispielsweise Starter oder Stabilisatoren und weitere Additive zu nennen. Ein Überblick über solche Komponenten gibt Plastics Additive Handbook, Hans Zweifel, Hanser Verlag, München (ISBN 3-446-21654-5). Auch hierbei kann alternativ oder zusätzlich zu dem Inkontaktbringen des Gases mit der erfindungsgemäßen Suspension die Oberfläche der Gegenstände mit der erfindungsgemäßen Suspension behandelt werden.

Der Geruchsstoff oder die Geruchsstoffe, die den Geruch erzeugen, können in dem Gas in gelöster Form vorliegen oder selbst gasförmig sein und somit einen "Bestandteil" eines Gasgemisches darstellen. Im Rahmen der vorliegenden Erfindung wird der Begriff "Geruchsstoff" ebenfalls vereinfachend verwendet, auch wenn es sich um ein Gemisch von mehreren Geruchsstoffen handelt. Geruchsstoffe sind hierbei Stoffe, die über den Geruchssinn des Menschen wahrgenommen werden können.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder Iod enthält oder ein ungesättigter oder aromatischer Kohlenwasserstoff oder ein gesättigter oder ungesättigter Aldehyd oder Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, zyklische oder azyklische Amine, Thiole, Thioether sowie Aldehyde, Ketone, Ester, Ether Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie zyklische oder azyklische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe handeln, die beispielsweise zur Herstellung von Parfümen verwendet werden. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskatellersalbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl 2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butyl cyclohexylacetat, Anisylacetate, Allylcyclohexyloxyacetat, Ethyllinalool, Eugenol, Coumarin, Ethyl acetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butylalpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300°C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250°C, mehr bevorzugt weniger als 230°C, insbesondere bevorzugt weniger als 200°C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20°C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20°C) auf, mehr bevorzugt einen Dampfdruck von mehr als 0,05 kPa (20°C) auf. Besonders bevorzugt weisen die Geruchsstoffe einen Dampfdruck von mehr als 0,1 kPa (20°C) auf.

Das poröse metallorganische Gerüstmaterial, das in der erfindungsgemäßen Suspension enthalten ist, enthält mindestens eine an mindestens ein Metallion koordinativ gebundene mindestens zweizähnige organische Verbindung. Dieses metallorganische Gerüstmaterial (MOF) wird beispielsweise beschrieben in US 5,648,508, EP-A-0 790 253, M. O-Keeffe et al., J. Sol. State Chem., 152 (2000), Seite 3 bis 20, H. Li et al., Nature 402, (1999), Seite 276, M. Eddaoudi et al., Topics in Catalysis 9, (1999), Seite 105 bis 111, B. Chen et al., Science 291, (2001), Seite 1021 bis 1023 und DE-A-101 11 230.

Die MOF's gemäß der vorliegenden Erfindung enthalten Poren, insbesondere Mirko- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie Pure Applied Chem. 45, Seite 71, insbesondere auf Seite 79 (1976) angegeben ist. Die Anwesenheit von Mikro- und/oder Mesoporen kann mit Hilfe von Sorptionsmessungen überprüft werden, wobei diese Messungen die Aufnahmekapazität der MOF für Stickstoff bei 77 Kelvin gemäß DIN 66131 und/oder DIN 66134 bestimmt.

Vorzugsweise beträgt die spezifische Oberfläche - berechnet nach dem Langmuir-Modell (DIN 66131, 66134) für ein MOF in Pulverform bei mehr als 5 m²/g, mehr bevorzugt über 10 m²/g, mehr bevorzugt mehr als 50 m²/g, weiter mehr bevorzugt mehr als 500 m²/g, weiter mehr bevorzugt mehr als 1000 m²/g und besonders bevorzugt mehr als 1500 m²/g.

Die Metallkomponente im Gerüstmaterial nach der vorliegenden Erfindung ist vorzugsweise ausgewählt aus den Gruppen Ia, IIa, IIIa, IVa bis VIIIa und Ib bis VIb. Besonders bevorzugt sind Mg, Ca, Sr, Ba, Sc, Y, Ti, Zr, Hf, V, Nb, Ta, Cr, Mo, W, Mn, Re, Fe, Ro, Os, Co, Rh, Ir, Ni, Pd, Pt, Cu, Ag, Au, Zn, Cd, Hg, Al, Ga, In, TI, Si, Ge, Sn, Pb, As, Sb und Bi. Mehr bevorzugt sind Zn, Cu, Ni, Pd, Pt, Ru, Rh und Co. Insbesondere bevorzugt Zn, Al, Ni und Cu. In Bezug auf die Ionen dieser Elemente sind besonders zu erwähnen Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺ W³⁺ Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga3^{+,} In³⁺, Tl³⁺, Si⁴⁺, Si²⁺, Ge4⁺, Ge²⁺, Sn4^{+,} Sn^{2+,} Pb⁴⁺, Pb²⁺, As⁵⁺, As³⁺ As⁺, Sb⁵⁺ Sb³⁺, Sb⁺, Bi⁵⁺, Bi³⁺ und Bi⁺.

Der Begriff "mindestens zweizähnige organische Verbindung" bezeichnet eine organische Verbindung, die mindestens eine funktionelle Gruppe enthält, die in der Lage ist, zu einem gegebenen Metallion mindestens zwei, bevorzugt zwei koordinative Bindungen, und/oder zu zwei oder mehr, bevorzugt zwei Metallatomen jeweils eine koordinative Bindung auszubilden.

Als funktionelle Gruppen, über die die genannten koordinativen Bindungen ausgebildet werden kann, sind insbesondere beispielsweise folgende funktionellen Gruppen zu nennen: -CO₂H, -CS₂H, -NO₂, -B(OH)₂, -SO₃H -Si(OH)₃, -Ge(OH)₃, -Sn(OH)₃, -Si(SH)₄, -Ge(SH)₄, -Sn(SH)₃, -PO₃H, -AsO₃H -AsO₄H -P(SH)₃, -As(SH)₃, -CH(RSH)₂, -C(RSH)₃> -CH(RNH₂)₂> -C(RNH₂)₃, -CH(ROH)₂, -C(ROH)₃, -CH(RCN)₂, -C(RCN)₃> wobei R beispielsweise bevorzugt eine Alkylengruppe mit 1, 2, 3, 4 oder 5 Kohlenstoffatomen wie beispielsweise eine Methylen-, Ethylen-, n-Propylen-, i-Propylen, n-Butylen-, i-Butylen-, tert-Butylen- oder n-Pentylengruppe, oder eine Arylgruppe, enthaltend 1 oder 2 aromatische Kerne wie beispielsweise 2 C₆-Ringe, die gegebenenfalls kondensiert sein können und unabhängig voneinander mit mindestes jeweils einem Substituenten geeignet substituiert sein können, und/oder die unabhängig voneinander jeweils mindestens ein Heteroatom wie beispielsweise N, O und/oder S enthalten können. Gemäß ebenfalls bevorzugter Ausführungsformen sind funktionelle Gruppen zu nennen, bei denen der oben genannte Rest R nicht vorhanden ist. Diesbezüglich sind unter anderem -CH(SH)₂, -C(SH)₃, -CH(NH₂)₂, -C(NH₂)₃, -CH(OH)₂, -C(OH)₃, -CH(CN)₂ oder -C(CN)₃ zu nennen.

Die mindestens zwei funktionellen Gruppen können grundsätzlich an jede geeignete organische Verbindung gebunden sein, solange gewährleistet ist, dass die diese funktionellen Gruppen aufweisende organische Verbindung zur Ausbildung der koordinativen Bindung und zur Herstellung des Gerüstmaterials befähigt ist.

Bevorzugt leiten sich die organischen Verbindungen, die die mindestens zwei funktionellen Gruppen enthalten, von einer gesättigten oder ungesättigten aliphatischen Verbindung oder einer aromatischen Verbindung oder einer sowohl aliphatischen als auch aromatischen Verbindung ab.

Die aliphatische Verbindung oder der aliphatische Teil der sowohl aliphatischen als auch aromatischen Verbindung kann linear und/oder verzweigt und/oder cyclisch sein, wobei auch mehrere Cyclen pro Verbindung möglich sind. Weiter bevorzugt enthält die aliphatische Verbindung oder der aliphatische Teil der sowohl aliphatischen als auch aromatischen Verbindung 1 bis 15, weiter bevorzugt 1 bis 14, weiter bevorzugt 1 bis 13, weiter bevorzugt 1 bis 12, weiter bevorzugt 1 bis 11 und insbesondere bevorzugt 1 bis 10 C-Atome wie beispielsweise 1, 2, 3, 4, 5, 6, 7, 8, 9 oder 10 C-Atome. Insbesondere bevorzugt sind hierbei unter anderem Methan, Adamantan, Acetylen, Ethylen oder Butadien.

Die aromatische Verbindung oder der aromatische Teil der sowohl aromatischen als auch aliphatischen Verbindung kann einen oder auch mehrere Kerne wie beispielsweise zwei, drei, vier oder fünf Kerne aufweisen, wobei die Kerne getrennt voneinander und/oder mindestens zwei Kerne in kondensierter Form vorliegen können. Besonders bevorzugt weist die aromatische Verbindung oder der aromatische Teil der sowohl aliphatischen als auch aromatischen Verbindung einen, zwei oder drei Kerne auf, wobei einer oder zwei Kerne besonders bevorzugt sind. Unabhängig voneinander kann weiter jeder Kern der genannten Verbindung mindestens ein Heteroatom wie beispielsweise N, O, S, B, P, Si, Al, bevorzugt N, O und/oder S enthalten. Weiter bevorzugt enthält die aromatische Verbindung oder der aromatische Teil der sowohl aromatischen als auch aliphatischen Verbindung einen oder zwei C₆-Kerne, wobei die zwei entweder getrennt voneinander oder in kondensierter Form vorliegen. Insbesondere sind als aromatische Verbindungen Benzol, Naphthalin und/oder Biphenyl und/oder Bipyridyl und/oder Pyridyl zu nennen.

Beispielsweise sind unter anderem trans-Muconsäure oder Fumarsäure oder Phenylenbisacrylsäure zu nennen.

Beispielsweise sind im Rahmen der vorliegenden Erfindung Dicarbonsäuren wie etwa Oxalsäure, Bernsteinsäure, Weinsäure, 1,4-Butandicarbonsäure, 4-Oxo-Pyran-2,6-dicarbonsäure, 1,6-Hexandicarbonsäure, Decandicarbonsäure, 1,8-Heptadecandicarbonsäure, 1,9-Heptadecandicarbonsäure, Heptadecandicarbonsäure, Acetylendicarbonsäure, 1,2-Benzoldicarbonsäure, 2,3-Pyridindicarbonsäure, Pyridin-2,3-dicarbonsäure, 1,3-Butadien-1,4-dicarbonsäure, 1,4-Benzoldicarbonsäure, p-Benzoldicarbonsäure, Imidazol-2,4-dicarbonsäure, 2-Methyl-chinolin-3,4-dicarbonsäure, Chinolin-2,4-dicarbonsäure, Chinoxalin-2,3-dicarbonsäure, 6-Chlorchinoxalin-2,3-dicarbonsäure, 4,4'-Diaminphenylmethan-3,3'-dicarbonsäure, Chinolin-3,4-dicarbonsäure, 7-Chlor-4-hydroxychinolin-2,8-dicarbonsäure, Diimiddicarbonsäure, Pyridin-2,6-dicarbonsäure, 2-Methylimidazol-4,5-dicarbonsäure, Thiophen-3,4-dicarbonsäure, 2-Isopropylimidazol-4,5-dicarbonsäure, Tetrahydropyran-4,4-dicarbonsäure, Perylen-3,9-dicarbonsäure, Perylendicarbonsäure, Pluriol E 200-dicarbonsäure, 3,6-Dioxaoctandicarbonsäure, 3,5-Cyclohexadien-1,2-dicarbonsäure, Octadicarbonsäure, Pentan-3,3-carbonsäure, 4,4'-Diamino-1,1'-diphenyl-3,3'-dicarbon-säure, 4,4'-Diaminodiphenyl-3,3'-dicarbonsäure, Benzidin-3,3'-dicarbonsäure, 1,4-bis-(Phenylamino)-benzol-2,5-dicarbonsäure, 1 J'-Dinaphthyl-S.S'-dicarbonsäure, 7-Chlor-8-methylchinolin-2,3-dicarbonsäure, 1-Anilino-anthrachinon-2,4'-dicarbonsäure, Poly-tetrahydrofuran-250-dicarbonsäure, 1,4-bis-(Carboxymethyl)-piperazin-2,3-dicarbon-säure, 7-Chlorchinolin-3,8-dicarbonsäure, 1-(4-Carboxy)-phenyl-3-(4-chlor)-phenylpyrazolin-4,5-dicarbonsäure, 1,4,5,6,7,7,-Hexachlor-5-norbornen-2,3-dicarbonsäure, Phenylindandicarbonsäure, 1,3-Dibenzyl-2-oxo-imidazolidin-4,5-dicarbonsäure, 1,4-Cyclohexandicarbonsäure, Naphthalin-1,8-dicarbonsäure, 2-Benzoylbenzol-1,3-dicarbonsäure, 1,3-Dibenzyl-2-oxoimidazolidin-4,5-cis-dicarbonsäure, 2,2'-Bichinolin-4,4'-dicarbonsäure, Pyridin-3,4-dicarbonsäure, 3,6,9-Trioxaundecandicarbonsäure, O-Hydroxybenzophenondicarbonsäure, Pluriol E 300-dicarbonsäure, Pluriol E 400-dicarbonsäure, Pluriol E 600-dicarbonsäure, Pyrazol-3,4-dicarbonsäure, 2,3-Pyrazindicarbonsäure, 5,6-Dimethyl-2,3-pyrazindicarbonsäure, 4,4'-Diaminodiphenyletherdiimiddicarbonsäure, 4,4'-Diaminodiphenylmethandiimiddicarbonsäure, 4,4'-Diaminodiphenylsulfondiimiddicarbonsäure, 2,6-Naphthalindicarbonsäure, 1,3-Adamantandicarbonsäure, 1,8-Naphthalindicarbonsäure, 2,3-Naphthalindicarbonsäure, 8-Methoxy-2,3-naphthalindicarbonsäure, 8-Nitro-2,3-naphthalincarbonsäure, 8-Sulfo-2,3-naphthalindicarbonsäure, Anthracen-2,3-dicarbonsäure, 2',3'-Diphenyl-p-terphenyl-4,4"-dicarbonsäure, Diphenylether-4,4'-dicarbonsäure, Imidazol-4,5-dicarbonsäure, 4(1 H)-Oxothiochromen-2,8-dicarbonsäure, 5-tert-Butyl -1,3-benzoldicarbonsäure, 7,8-Chinolindicarbonsäure, 4,5-Imidazoldicarbonsäure, 4-Cyclohexen-1,2-dicarbonsäure, Hexatriacontandicarbonsäure, Tetradecandicarbonsäure, 1,7-Heptadicarbonsäure, 5-Hydroxy-1,3-Benzoldicarbonsäure, Pyrazin-2,3-dicarbonsäure, Furan-2,5-dicarbonsäure, 1-Nonen-6,9-dicarbonsäure, Eicosendicarbonsäure, 4,4'-Dihydroxydiphenylmethan-3,3'-dicarbonsäure, 1-Amino-4-methyl-9,10-dioxo-9,10-dihydroanthracen-2,3-dicarbonsäure, 2,5-Pyridindicarbonsäure, Cyclohexen-2,3-dicarbonsäure,2,9-Dichlorfluorubin-4,11-dicarbonsäure, 7-Chlor-3-mtehylchinolin-6,8-dicarbonsäure, 2,4-Dichlorbenzophenon-2',5'-dicarbonsäure, 1,3-benzoldicarbonsäure, 2,6-Pyridindicarbonsäure, 1-Methylpyrrol-3,4-dicarbonsäure, 1-Benzyl-1H-pyrrol-3,4-dicarbonsäure, Anthrachinon-1,5-dicarbonsäure, 3,5-Pyrazoldicarbonsäure, 2-Nitrobenzol-1,4-dicarbonsäure, Heptan-1,7-dicarbonsäure, Cyclobutan-1,1-dicarbonsäure 1,14-Tetradecandicarbonsäure, 5,6-Dehydronorbornan-2,3-dicarbonsäure oder 5-Ethyl-2,3-Pyridindicarbonsäure,
Tricarbonsäuren wie etwa
2-Hydroxy-1,2,3-propantricarbonsäure, 7-Chlor-2,3,8-chinolintricarbonsäure, 1,2,4-Benzoltricarbonsäure, 1,2,4-Butantricarbonsäure, 2-Phosphono-1,2,4-butantricarbonsäure, 1,3,5-Benzoltricarbonsäure, 1-Hydroxy-1,2,3-Propantricarbonsäure, 4,5-Dihydro-4,5-dioxo-1H-pyrrolo[2,3-F]chinolin-2,7,9-tricarbonsäure, 5-Acetyl-3-amino-6-methylbenzol-1,2,4-tricarbonsäure, 3-Amino-5-benzoyl-6-methylbenzol-1,2,4-tricarbonsäure, 1,2,3-Propantricarbonsäure oder Aurintricarbonsäure,
oder Tetracarbonsäuren wie etwa
1,1-Dioxidperylo[1,12-BCD]thiophen-3,4,9,10-tetracarbonsäure, Perylentetracarbonsäuren wie Perylen-3,4,9,10-tetracarbonsäure oder Perylen-1,12-sulfon-3,4,9,10-tetracarbonsäure, Butantetracarbonsäuren wie 1,2,3,4-Butantetracarbonsäure oder Meso-1,2,3,4-Butantetracarbonsäure, Decan-2,4,6,8-tetracarbonsäure, 1,4,7,10,13,16-Hexaoxacyclooctadecan-2,3,11,12-tetracarbonsäure, 1,2,4,5-Benzoltetracarbonsäure, 1,2,11,12-Dodecantetracarbonsäure, 1,2,5,6-Hexantetracarbonsäure, 1,2,7,8-Octantetracarbonsäure, 1,4,5,8-Naphthalintetracarbonsäure, 1,2,9,10-Decantetracarbonsäure, Benzophenontetracarbonsäure, 3,3',4,4'-Benzophenontetracarbonsäure, Tetrahydrofurantetracarbonsäure oder Cyclopentantetracarbonsäuren wie Cyclopentan-1,2,3,4-tetracarbonsäure zu nennen.

Ganz besonders bevorzugt werden gegebenenfalls mindestens einfach substituierte mono-, di-, tri-, tetra- oder höherkernige aromatische Di-, Tri- oder Tetracarbonsäuren eingesetzt, wobei jeder der Kerne mindestens ein Heteroatom enthalten kann, wobei zwei oder mehr Kerne gleiche oder unterschiedliche Heteroatome enthalten kann. Beispielsweise bevorzugt werden monokernige Dicarbonsäuren, monokernige Tricarbonsäuren, monokernige Tetracarbonsäuren, dikernige Dicarbonsäuren, dikernige Tricarbonsäuren, dikernige Tetracarbonsäuren, trikernige Dicarbonsäuren, trikernige Tricarbonsäuren, trikernige Tetracarbonsäuren, tetrakernige Dicarbonsäuren, tetrakernige Tricarbonsäuren und/oder tetrakernige Tetracarbonsäuren. Geeignete Heteroatome sind beispielsweise N, O, S, B, P, Si, Al, bevorzugte Heteroatome sind hierbei N, S und/oder O. Als geeigneter Substituent ist diesbezüglich unter anderem -OH, eine Nitrogruppe, eine Aminogruppe oder eine Alkyl- oder Alkoxygruppe zu nennen.

Insbesondere bevorzugt werden als mindestens zweizähnige organische Verbindungen Acetylendicarbonsäure (ADC), Benzoldicarbonsäuren, Naphthalindicarbonsäuren, Biphenyldicarbonsäuren wie beispielsweise 4,4'-Biphenyldicarbonsäure (BPDC), Bipyridindicarbonsäuren wie beispielsweise 2,2'-Bipyridindicarbonsäuren wie beispielsweise 2,2'-Bipyridin-5,5'-dicarbonsäure, Benzoltricarbonsäuren wie beispielsweise 1,2,3-Benzoltricarbonsäure oder 1,3,5-Benzoltricarbonsäure (BTC), Adamantantetracarbonsäure (ATC), Adamantandibenzoat (ADB) Benzoltribenzoat (BTB), Methantetrabenzoat (MTB), Adamantantetrabenzoat oder Dihydroxyterephthalsäuren wie beispielsweise 2,5-Dihydroxyterephthalsäure (DHBDC) eingesetzt.

Ganz besonders bevorzugt werden unter anderem Isophtalsäure, Terephthalsäure, 2,5-Dihydroxyterephthalsäure, 1,2,3-Benzoltricarbonsäure, 1,3,5-Benzoltricarbonsäure oder 2,2'-Bipyridin-5,5'-dicarbonsäure eingesetzt.

Neben diesen mindestens zweizähnigen organischen Verbindungen kann der MOF auch eine oder mehrere einzähnige Liganden umfassen.

Geeignete Lösemittel zur Herstellung der MOF sind unter anderem Ethanol, Dimethylformamid, Toluol, Methanol, Chlorbenzol, Diethylformamid, Dimethylsulfoxid, Wasser, Wasserstoffperoxid, Methylamin, Natronlauge, N-Methylpolidonether, Acetonitril, Benzylchlorid, Triethylamin, Ethylenglykol und Gemische hiervon. Weitere Metallionen, mindestens zweizähnige organische Verbindungen und Lösemittel für die Herstellung von MOF sind unter anderem in US-A 5,648,508 oder DE-A 101 11 230 beschrieben.

Die Porengröße des MOF kann durch Wahl des geeigneten Liganden und/oder der mindestens zweizähnigen organischen Verbindung gesteuert werden. Allgemein gilt, dass je größer die organische Verbindung desto größer die Porengröße ist. Vorzugsweise beträgt die Porengröße von 0,2 nm bis 30 nm, besonders bevorzugt liegt die Porengröße im Bereich von 0,3 nm bis 3 nm bezogen auf das kristalline Material.

Nachfolgend sind Beispiele für MOF's angegeben. Neben der Kennzeichnung des MOF, dem Metall sowie dem mindestens zweizähnigen Liganden ist weiterhin das Lösemittel sowie die Zellenparameter (Winkel α, β und γ sowie die Abstände A, B und C in Å) angegeben. Letztere wurden durch Röntgenbeugung bestimmt.

| **MOF-n** | **Inhaltsstoffe molares Verhältnis M+L** | **Solvens s** | α | β | γ | **a** | **b** | **c** | **Raumgruppe** |
|---|---|---|---|---|---|---|---|---|---|
| MOF-0 | Zn(NO₃)₂-6H₂O | Ethanol | 90 | 90 | 120 | 16.711 | 16.711 | 14.189 | P6(3)/ |
| | H₃(BTC) | | | | | | | | Mcm |
| MOF-2 | Zn(NO₃)₂·6H₂O | DMF | 90 | 102.8 | 90 | 6.718 | 15.49 | 12.43 | P2(1)/n |
| | (0.246 mmol) | Toluen | | | | | | | |
| | H₂(BDC) | | | | | | | | |
| | 0.241 mmol) | | | | | | | | |
| MOF-3 | Zn(NO₃)₂-6H₂O | DMF | 99.72 | 111.11 | 108.4 | 9.726 | 9.911 | 10.45 | P-1 |
| | (1.89 mmol) | MeOH | | | | | | | |
| | H₂(BDC) | | | | | | | | |
| | (1.93 mmol) | | | | | | | | |
| MOF-4 | Zn(NO₃)₂-6H₂O | Ethanol | 90 | 90 | 90 | 14.728 | 14.728 | 14.728 | P2(1)3 |
| | (1.00 mmol) | | | | | | | | |
| | H₃(BTC) | | | | | | | | |
| | (0.5 mmol) | | | | | | | | |
| MOF-5 | Zn(NO₃)₂-6H₂O | DMF | 90 | 90 | 90 | 25.669 | 25.669 | 25.669 | Fm-3m |
| | (2.22 mmol) | Chlor- | | | | | | | |
| | H₂(BDC) | benzen | | | | | | | |
| | (2.17 mmol) | | | | | | | | |
| MOF-38 | Zn(NO₃)₂-6H₂O | DMF | 90 | 90 | 90 | 20.657 | 20.657 | 17.84 | 14cm |
| | (0.27 mmol) | Chlor- | | | | | | | |
| | H₃(BTC) | benzen | | | | | | | |
| | (0.15 mmol) | | | | | | | | |
| MOF-31 | Zn(NO₃)₂-6H₂O | Ethanol | 90 | 90 | 90 | 10.821 | 10.821 | 10.821 | Pn(-3)m |
| Zn(ADC)₂ | 0.4 mmol | | | | | | | | |
| | H₂(ADC) | | | | | | | | |
| | 0.8 mmol | | | | | | | | |
| MOF-12 | Zn(NO₃)₂-6H₂O | Ethanol | 90 | 90 | 90 | 15.745 | 16.907 | 18.167 | Pbca |
| Zn₂(ATC) | 0.3 mmol | | | | | | | | |
| | H₄(ATC) | | | | | | | | |
| | 0.15 mmol | | | | | | | | |
| MOF-20 | Zn(NO₃)₂-6H₂O | DMF | 90 | 92.13 | 90 | 8.13 | 16.444 | 12.807 | P2(1)/c |
| ZnNDC | 0.37 mmol | Chlor- | | | | | | | |
| | H₂NDC | benzen | | | | | | | |
| | 0.36 mmol | | | | | | | | |
| MOF-37 | Zn(NO₃)₂·6H₂O | DEF | 72.38 | 83.16 | 84.33 | 9.952 | 11.576 | 15.556 | P-1 |
| | 0.2 mmol | Chlor- | | | | | | | |
| | H₂NDC | benzen | | | | | | | |
| | 0.2 mmol | | | | | | | | |
| MOF-8 | Tb(NO₃)₃-5H₂O | DMSO | 90 | 115.7 | 90 | 19.83 | 9.822 | 19.183 | C2/c |
| Tb₂(ADC) | 0.10 mmol | MeOH | | | | | | | |
| | H₂ADC | | | | | | | | |
| | 0.20 mmol | | | | | | | | |
| MOF-9 | Tb(NO₃)₃·5H₂O | DMSO | 90 | 102.09 | 90 | 27.056 | 16.795 | 28.139 | C2/c |
| Tb₂ (ADC) | 0.08 mmol | | | | | | | | |
| | H₂ADB | | | | | | | | |
| | 0.12 mmol | | | | | | | | |
| MOF-6 | Tb(NO₃)₃.5H₂O | DMF | 90 | 91.28 | 90 | 17.599 | 19.996 | 10.545 | P21/c |
| | 0.30 mmol | MeOH | | | | | | | |
| | H₂ (BDC) | | | | | | | | |
| | 0.30 mmol | | | | | | | | |
| MOF-7 | Tb(NO₃)₃·5H₂O | H₂O | 102.3 | 91.12 | 101.5 | 6.142 | 10.069 | 10.096 | P-1 |
| | 0.15 mmol | | | | | | | | |
| | H₂(BDC) | | | | | | | | |
| | 0.15 mmol | | | | | | | | |
| MOF-69A | Zn(NO₃)₂·6H₂O | DEF | 90 | 111.6 | 90 | 23.12 | 20.92 | 12 | C2/c |
| | 0.083 mmol | H₂O₂ | | | | | | | |
| | 4,4'BPDC | MeNH₂ | | | | | | | |
| | 0.041 mmol | | | | | | | | |
| MOF-69B | Zn(NO₃)₂·6H₂O | DEF | 90 | 95.3 | 90 | 20.17 | 18.55 | 12.16 | C2/c |
| | 0.083 mmol | H₂O₂ | | | | | | | |
| | 2,6-NCD | MeNH₂ | | | | | | | |
| | 0.041 mmol | | | | | | | | |
| MOF-11 | Cu(NO₃)₂·2.5H₂O | H₂O | 90 | 93.86 | 90 | 12.987 | 11.22 | 11.336 | C2/c |
| Cu₂(ATC) | 0.47 mmol | | | | | | | | |
| | H₂ATC | | | | | | | | |
| | 0.22 mmol | | | | | | | | |
| MOF-11 Cu₂(ATC) dehydr. | | | 90 | 90 | 90 | 8.4671 | 8.4671 | 14.44 | P42/ mmc |
| MOF-14 | Cu(NO₃)₂·2.5H₂O | H₂O | 90 | 90 | 90 | 26.946 | 26.946 | 26.946 | Im-3 |
| Cu₃ (BTB) | 0.28 mmol | DMF | | | | | | | |
| | H₃BTB | EtOH | | | | | | | |
| | 0.052 mmol | | | | | | | | |
| MOF-32 | Cd(NO₃)₂·4H₂O | H₂O | 90 | 90 | 90 | 13.468 | 13.468 | 13.468 | P(-4)3m |
| Cd(ATC) | 0.24 mmol | NaOH | | | | | | | |
| | H₄ATC | | | | | | | | |
| | 0.10 mmol | | | | | | | | |
| MOF-33 | ZnCl₂ | H₂O | 90 | 90 | 90 | 19.561 | 15.255 | 23.404 | Imma |
| Zn₂ (ATB) | 0.15 mmol | DMF | | | | | | | |
| | H₄ATB | EtOH | | | | | | | |
| | 0.02 mmol | | | | | | | | |
| MOF-34 Ni(ATC) | Ni(NO₃)₂·6H₂O 0.24 mmol H₄ATC 0.10 mmol | H₂O NaOH | 90 | 90 | 90 | 10.066 | 11.163 | 19.201 | P2₁2₁2₁ |
| MOF-36 Zn₂ (MTB) | Zn(NO₃)₂·4H₂O 0.20 mmol H₄MTB 0.04 mmol | H₂O DMF | 90 | 90 | 90 | 15.745 | 16.907 | 18.167 | Pbca |
| MOF-39 Zn₃O(HBT B) | Zn(NO₃)₂·4H₂O 0.27 mmol H₃BTB 0.07 mmol | H₂O DMF EtOH | 90 | 90 | 90 | 17.158 | 21.591 | 25.308 | Pnma |
| NO305 | FeCl₂4H₂O 5.03 mmol Ameisensäure. 86.90 mmol | DMF 90 | 90 | 90 | 120 | 8.2692 | 8.2692 | 63.566 | R-3c |
| NO306A | FeCl₂.4H₂O 5.03 mmol Ameisensäure. 86.90 mmol | DEF | 90 | 90 | 90 | 9.9364 | 18.374 | 18.374 | Pbcn |
| | | | | | | | | | |
| NO29 MOF-0 ähnlich | Mn(Ac)₂·4H₂O 0.46 mmol H₃BTC 0.69 mmol | DMF | 120 | 90 | 90 | 14.16 | 33.521 | 33.521 | P-1 |
| BPR48 A2 | Zn(NO₃)₂6H₂O 0.012 mmol H₂BDC 0.012 mmol | DMSO Toluen | 90 | 90 | 90 | 14.5 | 17.04 | 18.02 | Pbca |
| BPR69 B1 | Cd(NO₃)₂4H₂O 0.0212 mmol H₂BDC 0.0428 mmol | DMSO | 90 | 98.76 | 90 | 14.16 | 15.72 | 17.66 | Cc |
| BPR92 A2 | Co(NO₃)₂6H₂O 0.018 mmol H₂BDC 0.018 mmol | NMP | 106.3 | 107.63 | 107.2 | 7.5308 | 10.942 | 11.025 | P1 |
| BPR95 C5 | Cd(NO₃)₂4H₂O 0.012 mmol H₂BDC 0.36 mmol | NMP | 90 | 112.8 | 90 | 14.460 | 11.085 | 15.829 | P2(1)/n |
| Cu C₆H₄O₆ | Cu(NO₃)₂·2.5H₂O 0.370 mmol H₂BDC(OH)₂ 0.37 mmol | DMF Chlorbenzen | 90 | 105.29 | 90 | 15.259 | 14.816 | 14.13 | P2(1)/c |
| M(BTC) MOF-0 ähnlich | Co(SO₄) H₂O 0.055 mmol H₃BTC 0.037 mmol | DMF | wie MOF-0 | | | | | | |
| Tb(C₆H₄O₆) | Tb(NO₃)₃·5H₂O 0.370 mmol H₂(C₆H₄O₆) 0.56 mmol | DMF Chlorbenzen | 104.6 | 107.9 | 97.147 | 10.491 | 10.981 | 12.541 | P-1 |
| Zn (C₂O₄) | ZnCl₂ 0.370 mmol Oxalsäure 0.37 mmol | DMF Chlorbenzen | 90 | 120 | 90 | 9.4168 | 9.4168 | 8.464 | P(-3)1m |
| Co(CHO) | Co(NO₃)₂-5H₂O 0.043 mmol Ameisensäure 1.60 mmol | DMF | 90 | 91.32 | 90 | 11.328 | 10.049 | 14.854 | P2(1)/n |
| Cd(CHO) | Cd(NO₃)₂-4H₂O 0.185 mmol formic acid 0.185 mmol | DMF | 90 | 120 | 90 | 8.5168 | 8.5168 | 22.674 | R-3c |
| Cu(C₃H₂O₄) | Cu(NO₃)₂·2.5H₂O 0.043 mmol Malonsäure. 0.192 mmol | DMF | 90 | 90 | 90 | 8.366 | 8.366 | 11.919 | P43 |
| Zn₆ (NDC)₅ MOF-48 | Zn(NO₃)₂·6H₂O 0.097 mmol 14 NDC 0.069 mmol | DMF Chlorbenzen H₂O₂ | 90 | 95.902 | 90 | 19.504 | 16.482 | 14.64 | C2/m |
| MOF-47 | Zn(NO₃)₂6H₂O 0.185 mmol H₂(BDC[CH₃]₄) 0.185 mmol | | 90 | 92.55 | 90 | 11.303 | 16.029 | 17.535 | P2(1)/c |
| MO25 | Cu(NO₃)₂·2.5H ₂O 0.084 mmol BPhDC 0.085 mmol | | 90 | 112.0 | 90 | 23.880 | 16.834 | 18.389 | P2(1)/c |
| Cu-Thio | Cu(NO₃)₂·2.5H ₂O 0.084 mmol Thiophen Dicarbonsäure 0.085 mmol | | 90 | 113.6 | 90 | 15.4747 | 14.514 | 14.032 | P2(1)/c |
| CIBDC1 | Cu(NO₃)₂2.5H ₂O 0.084 mmol H₂(BDCCl₂) 0.085 mmol | | 90 | 105.6 | 90 | 14.911 | 15.622 | 18.413 | C2/c |
| MOF-101 | Cu(NO₃)₂·2.5H | DMF | 90 | 90 | 90 | 21.607 | 20.607 | 20.073 | Fm3m |
| | ₂O | | | | | | | | |
| | 0.084 mmol | | | | | | | | |
| | BrBDC | | | | | | | | |
| | 0.085 mmol | | | | | | | | |
| Zn₃(BTC) | ZnCl₂ | DMF | 90 | 90 | 90 | 26.572 | 26.572 | 26.572 | Fm-3m |
| ₂ | 0.033 mmol | EtOH | | | | | | | |
| | H₃BTC | Base | | | | | | | |
| | 0.033 mmol | zugegeben | | | | | | | |
| MOF-j | Co(CH₃CO₂)₂·4 | H₂O | 90 | 112.0 | 90 | 17.482 | 12.963 | 6.559 | C2 |
| | H₂O | | | | | | | | |
| | (1.65 mmol) | | | | | | | | |
| | H₃(BZC) | | | | | | | | |
| | (0.95 mmol) | | | | | | | | |
| MOF-n | Zn(NO₃)₂·6H₂O | Ethanol | 90 | 90 | 120 | 16.711 | 16.711 | 14.189 | P6(3)/mcm |
| | H₃ (BTC) | | | | | | | | |
| PbBDC | Pb(NO₃)2 | DMF | 90 | 102.7 | 90 | 8.3639 | 17.991 | 9.9617 | P2(1)/n |
| | (0.181 mmol) | Ethanol | | | | | | | |
| | H₂(BDC) | | | | | | | | |
| | (0.181 mmol) | | | | | | | | |
| Znhex | Zn(NO₃)₂·6H₂O | DMF | 90 | 90 | 120 | 37.1165 | 37.117 | 30.019 | P3(1)c |
| | (0.171 mmol) | p-Xylen | | | | | | | |
| | H₃BTB | Ethanol | | | | | | | |
| | (0.114 mmol) | | | | | | | | |
| AS16 | FeBr₂ | DMF | 90 | 90.13 | 90 | 7.2595 | 8.7894 | 19.484 | P2(1)c |
| | 0.927 mmol | anhydr. | | | | | | | |
| | H₂(BDC) | | | | | | | | |
| | 0.927 mmol | | | | | | | | |
| AS27-2 | FeBr₂ | DMF | 90 | 90 | 90 | 26.735 | 26.735 | 26.735 | Fm3m |
| | 0.927 mmol | anhydr. | | | | | | | |
| | H₃(BDC) | | | | | | | | |
| | 0.464 mmol | | | | | | | | |
| AS32 | FeCl₃ | DMF an- | 90 | 90 | 120 | 12.535 | 12.535 | 18.479 | P6(2)c |
| | 1.23 mmol | hydr. Etha- | | | | | | | |
| | H₂(BDC) | nol | | | | | | | |
| | 1.23 mmol | | | | | | | | |
| AS54-3 | FeBr₂ | DMF an- | 90 | 109.98 | 90 | 12.019 | 15.286 | 14.399 | C2 |
| | 0.927 | hydr. | | | | | | | |
| | BPDC | n-Propanol | | | | | | | |
| | 0.927 mmol | | | | | | | | |
| AS61-4 | FeBr₂ | Pyridin | 90 | 90 | 120 | 13.017 | 13.017 | 14.896 | P6(2)c |
| | 0.927 mmol | anhydr. | | | | | | | |
| | m-BDC | | | | | | | | |
| | 0.927 mmol | | | | | | | | |
| AS68-7 | FeBr₂ | DMF an- | 90 | 90 | 90 | 18.3407 | 10.036 | 18.039 | Pca2, |
| | 0.927 mmol | hydr. | | | | | | | |
| | m-BDC | Pyridin | | | | | | | |
| | 1.204 mmol | | | | | | | | |
| Zn(ADC) | Zn(NO₃)₂·6H₂O | DMF | 90 | 99.85 | 90 | 16.764 | 9.349 | 9.635 | C2/c |
| | 0.37 mmol | Chlorben | | | | | | | |
| | H₂(ADC) | zen | | | | | | | |
| | 0.36 mmol | | | | | | | | |
| MOF-12 | Zn(NO₃)₂-6H₂O | Ethanol | 90 | 90 | 90 | 15.745 | 16.907 | 18.167 | Pbca |
| Zn₂ | 0.30 mmol | | | | | | | | |
| (ATC) | H₄(ATC) | | | | | | | | |
| | 0.15 mmol | | | | | | | | |
| MOF-20 | Zn(NO₃)₂·6H₂O | DMF | 90 | 92.13 | 90 | 8.13 | 16.444 | 12.807 | P2(1)/c |
| ZnNDC | 0.37 mmol | Chlorben- | | | | | | | |
| | H₂NDC | zen | | | | | | | |
| | 0.36 mmol | | | | | | | | |
| MOF-37 | Zn(NO₃)₂·6H₂O | DEF | 72.38 | 83.16 | 84.33 | 9.952 | 11.576 | 15.556 | P-1 |
| | 0.20 mmol | Chlorben- | | | | | | | |
| | H₂NDC | zen | | | | | | | |
| | 0.20 mmol | | | | | | | | |
| Zn(NDC) (DMSO) | Zn(NO₃)₂·6H₂O | DMSO | 68.08 | 75.33 | 88.31 | 8.631 | 10.207 | 13.114 | P-1 |
| | H₂NDC | | | | | | | | |
| Zn(NDC) | Zn(NO₃)₂·6H₂O | | 90 | 99.2 | 90 | 19.289 | 17.628 | 15.052 | C2/c |
| | H₂NDC | | | | | | | | |
| Zn(HPD | Zn(NO₃)₂·4H₂O | DMF | 107.9 | 105.06 | 94.4 | 8.326 | 12.085 | 13.767 | P-1 |
| C) | 0.23 mmol | H₂O | | | | | | | |
| | H₂(HPDC) | | | | | | | | |
| | 0.05 mmol | | | | | | | | |
| Co(HPD C) | Co(NO₃)₂·6H₂ O | DMF H₂O/ Etha- | 90 | 97.69 | 90 | 29.677 | .9.63 | 7.981 | C2/c |
| | 0.21 mmol | nol | | | | | | | |
| | H₂ (HPDC) | | | | | | | | |
| | 0.06 mmol | | | | | | | | |
| Zn₃(PDC )2.5 | Zn(NO₃)₂·4H₂O | DMF/ ClBz | 79.34 | 80.8 | 85.83 | 8.564 | 14.046 | 26.428 | P-1 |
| | 0.17 mmol | H20/ TEA | | | | | | | |
| | H₂(HPDC) | | | | | | | | |
| | 0.05 mmol | | | | | | | | |
| Cd₂ (TPDC)2 | Cd(NO₃)₂·4H₂ O | Methanol/ CHP H₂O | 70.59 | 72.75 | 87.14 | 10.102 | 14.412 | 14.964 | P-1 |
| | 0.06 mmol | | | | | | | | |
| | H₂(HPDC) | | | | | | | | |
| | 0.06 mmol | | | | | | | | |
| Tb(PDC) | Tb(NO₃)₃·5H₂O | DMF | 109.8 | 103.61 | 100.14 | 9.829 | 12.11 | 14.628 | P-1 |
| 1.5 | 0.21 mmol | H₂O/ Etha- | | | | | | | |
| | H₂(PDC) | nol | | | | | | | |
| | 0.034 mmol | | | | | | | | |
| ZnDBP | Zn(NO₃)₂·6H₂O | MeOH | 90 | 93.67 | 90 | 9.254 | 10.762 | 27.93 | P2/n |
| | 0.05 mmol | | | | | | | | |
| | Dibenzylphosphat | | | | | | | | |
| | 0.10 mmol | | | | | | | | |
| Zn₃(BPDC) | ZnBr₂ | DMF | 90 | 102.76 | 90 | 11.49 | 14.79 | 19.18 | P21/n |
| | 0.021 mmol | | | | | | | | |
| | 4,4'BPDC | | | | | | | | |
| | 0.005 mmol | | | | | | | | |
| CdBDC | Cd(NO₃)₂·4H₂O | DMF | 90 | 95.85 | 90 | 11.2 | 11.11 | 16.71 | P21/n |
| | 0.100 mmol | Na₂SiO | | | | | | | |
| | H₂(BDC) 0.401 mmol | 3 (aq) | | | | | | | |
| | | | | | | | | | |
| Cd-mBDC | Cd(NO₃)₂·4H₂O | DMF | 90 | 101.1 | 90 | 13.69 | 18.25 | 14.91 | C2/c |
| | 0.009 mmol | MeNH₂ | | | | | | | |
| | H₂(mBDC) | | | | | | | | |
| | 0.018 mmol | | | | | | | | |
| Zn₄OBND | Zn(NO₃)₂·6H₂O | DEF | 90 | 90 | 90 | 22.35 | 26.05 | 59.56 | Fmmm |
| C | 0.041 mmol | MeNH₂ | | | | | | | |
| | BNDC | H₂O₂ | | | | | | | |
| Eu(TCA) | Eu(NO₃)₃·6H₂O | DMF | 90 | 90 | 90 | 23.325 | 23.325 | 23.325 | Pm-3n |
| | 0.14 mmol | Chlor- | | | | | | | |
| | TCA | benzen | | | | | | | |
| | 0.026 mmol | | | | | | | | |
| Tb(TCA) | Tb(NO₃)₃·6H₂O | DMF | 90 | 90 | 90 | 23.272 | 23.272 | 23.372 | Pm-3n |
| | 0.069 mmol | Chlor- | | | | | | | |
| | TCA | benzen | | | | | | | |
| | 0.026 mmol | | | | | | | | |
| Formate | Ce(NO₃)₃·6H₂O | H₂O | 90 | 90 | 120 | 10.668 | 10.667 | 4.107 | R-3m |
| | 0.138 mmol | Ethanol | | | | | | | |
| | Ameisensäure. | | | | | | | | |
| | 0.43 mmol | | | | | | | | |
| | FeCl₂.4H₂O | DMF | 90 | 90 | 120 | 8.2692 | 8.2692 | 63.566 | R-3c |
| | 5.03 mmol | | | | | | | | |
| | Ameisensre. | | | | | | | | |
| | 86.90 mmol | | | | | | | | |
| | FeCl_{2·}4H₂O | DEF | 90 | 90 | 90 | 9.9364 | 18.374 | 18.374 | Pbcn |
| | 5.03 mmol | | | | | | | | |
| | Ameisensäure. | | | | | | | | |
| | 86.90 mmol | | | | | | | | |
| | FeCl₂·4H₂O | DEF | 90 | 90 | 90 | 8.335 | 8.335 | 13.34 | P-31c |
| | 5.03 mmol | | | | | | | | |
| | Ameisensäure. | | | | | | | | |
| | 86.90 mmol | | | | | | | | |
| N0330 | FeCl₂·4H₂O | Form- | 90 | 90 | 90 | 8.7749 | 11.655 | 8.3297 | Pnna |
| | 0.50 mmol | amid | | | | | | | |
| | Ameisensre. | | | | | | | | |
| NO332 | FeCl₂·4H₂O | DIP | 90 | 90 | 90 | 10.031 | 18.808 | 18.355 | Pbcn |
| | 0.50 mmol | | | | | 3 | | | |
| | Ameisensäure. | | | | | | | | |
| | 8.69 mmol | | | | | | | | |
| | | | | | | | | | |
| NO333 | FeCl₂·4H₂O | DBF | 90 | 90 | 90 | 45.2754 | 23.861 | 12.441 | Cmcm |
| | 0.50 mmol | | | | | | | | |
| | Ameisensäure. | | | | | | | | |
| | 8.69 mmol | | | | | | | | |
| NO335 | FeCl₂·4H₂O | CHF | 90 | 91.372 | 90 | 11.5964 | 10.187 | 14.945 | P21/n |
| | 0.50 mmol | | | | | | | | |
| | Ameisensäure. | | | | | | | | |
| | 8.69 mmol | | | | | | | | |
| NO336 | FeCl₂·4H₂O | MFA | 90 | 90 | 90 | 11.7945 | 48.843 | 8.4136 | Pbcm |
| | 0.50 mmol | | | | | | | | |
| | Ameisensäure. | | | | | | | | |
| | 8.69 mmol | | | | | | | | |
| NO13 | Mn(Ac)₂·4H₂O | Ethanol | 90 | 90 | 90 | 18.66 | 11.762 | 9.418 | Pbcn |
| | 0.46 mmol | | | | | | | | |
| | Benzoesäure. | | | | | | | | |
| | 0.92 mmol | | | | | | | | |
| | Bipyridin | | | | | | | | |
| | 0.46 mmol | | | | | | | | |
| N029 | Mn(Ac)₂·4H₂O | DMF | 120 | 90 | 90 | 14.16 | 33.521 | 33.521 | P-1 |
| MOF-0 | 0.46 mmol | | | | | | | | |
| ähnlich | H₃BTC | | | | | | | | |
| | 0.69 mmol | | | | | | | | |
| Mn(hfac)₂ | Mn(Acl₂·4H₂O | Ether | 90 | 95.32 | 90 | 9.572 | 17.162 | 14.041 | C2/c |
| (O₂CC₆H₅) | 0.46 mmol | | | | | | | | |
| | Hfac | | | | | | | | |
| | 0.92 mmol | | | | | | | | |
| | Bipyridin | | | | | | | | |
| | 0.46 mmol | | | | | | | | |
| BPR43G2 | Zn(NO₃)₂·6H₂O | DMF | 90 | 91.37 | 90 | 17.96 | 6.38 | 7.19 | C2/c |
| | 0.0288 mmol | CH₃CN | | | | | | | |
| | H₂BDC | | | | | | | | |
| | 0.0072 mmol | | | | | | | | |
| BPR48A2 | Zn(NO₃)₂6H₂O | DMSO | 90 | 90 | 90 | 14.5 | 17.04 | 18.02 | Pbca |
| | 0.012 mmol | Toluen | | | | | | | |
| | H₂BDC | | | | | | | | |
| | 0.012 mmol | | | | | | | | |
| BPR49B1 | Zn(NO₃)₂ 6H₂O | DMSO | 90 | 91.172 | 90 | 33.181 | 9.824 | 17.884 | C2/c |
| | 0.024 mmol | Metha- | | | | | | | |
| | H₂BDC | nol | | | | | | | |
| | 0.048 mmol | | | | | | | | |
| BPR56E1 | Zn(NO₃)₂ 6H₂O 0.012 mmol H₂BDC 0.024 mmol | DMSO n-propanol | 90 | 90.096 | 90 | 14.5873 | 14.153 | 17.183 | P2(1)/n |
| BPR68D10 | Zn(NO₃)₂ 6H₂O 0.0016 mmol H₃BTC 0.0064 mmol | DMSO Benzen | 90 | 95.316 | 90 | 10.0627 | 10.17 | 16.413 | P2(1)/c |
| BPR69B1 | Cd(NO₃)₂ 4H₂O 0.0212 mmol H₂BDC 0.0428 mmol | DMSO | 90 | 98.76 | 90 | 14.16 | 15.72 | 17.66 | Cc |
| BPR73E4 | Cd(NO₃)₂ 4H₂O 0.006 mmol h₂BDC 0.003 mmol | DMSO Toluen | 90 | 92.324 | | | | | |
| BPR76D5 | Zn(NO₃)₂ 6H₂O 0.0009 mmol H₂BzPDC 0.0036 mmol | DMSO | 90 | 104.17 | | | | | |
| BPR80B5 | Cd(NO₃)₂·4H₂O 0.018 mmol H₂BDC 0.036 mmol | DMF | 90 | 115.11 | | | | | |
| BPR80H5 | Cd(NO₃)₂4H₂O 0.027 mmol H₂BDC 0.027 mmol | DMF | 90 | 119.06 | | | | | |
| BPR82C6 | Cd(NO₃)₂4H₂O 0.0068 mmol H₂BDC 0.202 mmol | DMP | 90 | 90 | | | | | |
| BPR86C3 | Co(NO₃)₂6H₂O 0.0025 mmol H₂BDC 0.075 mmol | DMF | 90 | 90 | | | | | |
| BPR86H6 | Cd(NO₃)·6H₂O 0.010 mmo H₂BDC 0.010 mmol | DMF | 90 | 89.69 | | | | | |
| | Co(NO₃)₂ 6H₂O | NMP | 90 | 107.63 | | | | | |
| BPR95A2 | Zn(NO₃)₂6H₂O 0.012 mmol H₂BDC 0.012 mmol | NMP | 90 | 102.9 | | | | | |
| CuC₆F₄O₄ | Cu(NO₃)₂·2.5H₂O 0.370 mmol H₂BDC(OH) ₂ 0.37 mmol | DMF Chlor- benzen | 90 | 98.834 | 90 | 10.9675 | 24.43 | 22.553 | P2(1)/n |
| Fe Formic | FeCl₂-4H₂O 0.370 mmol Ameisensäure. 0.37 mmol | DMF | 90 | 91.543 | 90 | 11.495 | 9.963 | 14.48 | P2(1)/n |
| Mg Formic | Mg(NO₃)₂·6H₂O 0.370 mmol Ameisensäure. 0.37 mmol | DMF | 90 | 91.359 | 90 | 11.383 | 9.932 | 14.656 | P2(1)/n |
| MgC₆H₄O₆ | Mg(NO₃)₂·6H₂O 0.370 mmol H₂BDC(OH)₂ 0.37 mmol | DMF | 90 | 96.624 | 90 | 17.245 | 9.943 | 9.273 | C2/c |
| Zn C₂H₄BDC MOF-38 | ZnCl₂ 0.44 mmol CBBDC 0.261 mmol | DMF | 90 | 94.714 | 90 | 7.3386 | 16.834 | 12.52 | P2(1)/n |
| MOF-49 | ZnCl₂ 0.44 mmol m-BDC 0.261 mmol | DMF CH₃CN | 90 | 93.459 | 90 | 13.509 | 11.984 | 27.039 | P2/c |
| MOF-26 | Cu(NO₃)₂·5H₂O 0.084 mmol DCPE 0.085 mmol | DMF | 90 | 95.607 | 90 | 20.8797 | 16.017 | 26.176 | P2(1)/n |
| MOF-112 | Cu(NO₃)₂·2.5H₂O 0.084 mmol *o*-Br-*m*-BDC 0.085 mmol | DMF Ethanol | 90 | 107.49 | 90 | 29.3241 | 21.297 | 18.069 | C2/c |
| MOF-109 | Cu(NO₃)₂·2.5H₂O 0.084 mmol KDB 0.085 mmol | DMF | 90 | 111.98 | 90 | 23.8801 | 16.834 | 18.389 | P2(1)/c |
| MOF-111 | Cu(NO₃)₂·2.5H₂O 0.084 mmol o-BrBDC 0.085 mmol | DMF Ethanol | 90 | 102.16 | 90 | 10.6767 | 18.781 | 21.052 | C2/c |
| MOF-110 | Cu(NO₃)₂·2.5H₂O 0.084 mmol Thiophen Dicarbonsäure 0.085 mmol | DMF | 90 | 90 | 120 | 20.0652 | 20.065 | 20.747 | R-3/m |
| MOF-107 | Cu(NO₃)₂·2.5H₂O 0.084 mmol Thiophen Dicarbonsäure 0.085 mmol | DEF | 104.8 | 97.075 | 95.206 | 11.032 | 18.067 | 18.452 | P-1 |
| MOF-108 | Cu(NO₃)₂·2.5H₂O 0.084 mmol Thiophen Dicarbonsäure 0.085 mmol | DBF/ Methanol | 90 | 113.63 | 90 | 15.4747 | 14.514 | 14.032 | C2/c |
| MOF-102 | Cu(NO₃)₂·2.5H₂O 0.084 mmol H₂(BDCCl₂) 0.085 mmol | DMF | 91.63 | 106.24 | 112.01 | 9.3845 | 10.794 | 10.831 | P-1 |
| Clbdc1 | Cu(NO₃)₂·2.5H₂O 0.084 mmol H₂(BDCCl₂) 0.085 mmol | DEF | 90 | 105.56 | 90 | 14.911 | 15.622 | 18.413 | P-1 |
| Cu(NMOP) | Cu(NO₃)₂·2.5H₂O 0.084 mmol NBDC 0.085 mmol | DMF | 90 | 102.37 | 90 | 14.9238 | 18.727 | 15.529 | P2(1)/m |
| Tb(BTC) | Tb(NO₃)₃·5H₂O 0.033 mmol H₃BTC 0.033 mmol | DMF | 90 | 106.02 | 90 | 18.6986 | 11.368 | 19.721 | |
| Zn₃(BTC)₂ Honk | ZnCl₂ 0.033 mmol H₃BTC 0.033 mmol | DMF Ethanol | 90 | 90 | 90 | 26.572 | 26.572 | 26.572 | Fm-3m |
| Zn₄O(NDC) | Zn(NO₃)₂·4H₂O 0.066 mmol 14NDC 0.066 mmol | DMF Ethanol | 90 | 90 | 90 | 41.5594 | 18.818 | 17.574 | aba2 |
| CdTDC | Cd(NO₃)₂·4H₂O 0.014 mmol Thiophen 0.040 mmol DABCO 0.020 mmol | DMF H₂O | 90 | 90 | 90 | 12.173 | 10.485 | 7.33 | Pmma |
| IRMOF-2 | Zn(NO₃)₂·4H₂O 0.160 mmol o-Br-BDC 0.60 mmol | DEF | 90 | 90 | 90 | 25.772 | 25.772 | 25.772 | Fm-3m |
| IRMOF-3 | Zn(NO₃)₂·4H₂O 0.20 mmol H₂ N-BDC 0.60 mmol | DEF Ethanol | 90 | 90 | 90 | 25.747 | 25.747 | 25.747 | Fm-3m |
| IRMOF-4 | Zn(NO₃)₂·4H₂O 0.11 mmol [C₃H₇O]₂-BDC 0.48 mmol | DEF | 90 | 90 | 90 | 25.849 | 25.849 | 25.849 | Fm-3m |
| IRMOF-5 | Zn(NO₃)₂·4H₂O 0.13 mmol [C₅H₁₁O]₂-BDC 0.50 mmol | DEF | 90 | 90 | 90 | 12.882 | 12.882 | 12.882 | Pm-3m |
| IRMOF-6 | Zn(NO₃)₂·4H₂O 0.20 mmol [C₂H₄]-BDC 0.60 mmol | DEF | 90 | 90 | 90 | 25.842 | 25.842 | 25.842 | Fm-3m |
| IRMOF-7 | Zn(NO₃)₂·4H₂O 0.07 mmol 1,4NDC 0.20 mmol | DEF | 90 | 90 | 90 | 12.914 | 12.914 | 12.914 | Pm-3m |
| IRMOF-8 | Zn(NO₃)₂·4H₂O 0.55 mmol 2,6NDC 0.42 mmol | DEF | 90 | 90 | 90 | 30.092 | 30.092 | 30.092 | Fm-3m |
| IRMOF-9 | Zn(NO₃)₂·4H₂O 0.05 mmol BPDC 0.42 mmol | DEF | 90 | 90 | 90 | 17.147 | 23.322 | 25.255 | Pnnm |
| IRMOF-10 | Zn(NO₃)₂·4H₂O 0.02 mmol BPDC 0.012 mmol | DEF | 90 | 90 | 90 | 34.281 | 34.281 | 34.281 | Fm-3m |
| IRMOF-11 | Zn(NO₃)₂·4H₂O 0.05 mmol HPDC 0.20 mmol | DEF | 90 | 90 | 90 | 24.822 | 24.822 | 56.734 | R-3m |
| IRMOF-12 | Zn(NO₃)₂·4H₂O 0.017 mmol HPDC 0.12 mmol | DEF | 90 | 90 | 90 | 34.281 | 34.281 | 34.281 | Fm-3m |
| | | | | | | | | | |
| IRMOF-13 | Zn(NO₃)₂·4H₂O 0.048 mmol PDC 0.31 mmol | DEF | 90 | 90 | 90 | 24.822 | 24.822 | 56.734 | R-3m |
| IRMOF-14 | Zn(NO₃)₂·4H₂O 0.17 mmol PDC 0.12 mmol | DEF | 90 | 90 | 90 | 34.381 | 34.381 | 34.381 | Fm-3m |
| IRMOF-15 | Zn(NO₃)₂·4H₂O 0.063 mmol TPDC 0.025 mmol | DEF | 90 | 90 | 90 | 21.459 | 21.459 | 21.459 | Im-3m |
| IRMOF-16 | Zn(NO₃)₂·4H₂O 0.0126 mmol TPDC 0.05 mmol | DEF NMP | 90 | 90 | 90 | 21.49 | 21.49 | 21.49 | Pm-3m |

- ADC: Acetylendicarbonsäure
- NDC: Naphthalindicarbonsäure
- BDC: Benzoldicarbonsäure
- ATC: Adamantantetracarbonsäure
- BTC: Benzoltricarbonsäure
- BTB: Benzoltribenzoesäure
- MTB: Methantetrabenzoesäure
- ATB: Adamantantetrabenzoesäure
- ADB: Adamantandibenzoesäure

Weitere MOF sind MOF-177, MOF-178, MOF-74, MOF-235, MOF-236, MOF-69 bis 80, MOF-501, MOF-502, welche in der Literatur beschrieben sind.

Insbesondere bevorzugt ist ein poröses metallorganisches Gerüstmaterial, bei dem Zn, Al, Ni oder Cu als Metallion und die mindestens zweizähnige organische Verbindung Terephtalsäure, Isophtalsäure, 2,6-Naphthalindicarbonsäure oder 1,3,5-Benzoltricarbonsäure ist.

Neben der konventionellen Methode zur Herstellung der MOF, wie sie beispielsweise in US 5,648,508 beschrieben ist, können diese auch auf elektrochemischem Wege hergestellt werden. Diesbezüglich wird auf DE-A 103 55 087 sowie WO-A 2005/049892 verwiesen. Die auf diesem Weg hergestellten MOFs weisen besonders gute Eigenschaften in Zusammenhang mit der Adsorption und Desorption von chemischen Stoffen, insbesondere von Gasen. Sie unterscheiden sich somit von denen, die konventionell hergestellt werden, auch wenn diese aus den gleichen organischen und Metallionenbestandteilen gebildet werden und sind daher als neue Gerüstmaterialien zu betrachten. Im Rahmen der vorliegenden Erfindung sind elektrochemisch hergestellte MOFs besonders bevorzugt.

Demgemäß betrifft die elektrochemischen Herstellung ein kristallines poröses metallorganischen Gerüstmaterial, enthaltend mindestens eine an mindestens ein Metallion koordinativ gebundene, mindestens zweizähnige organische Verbindung, welches in einem Reaktionsmedium, enthaltend die mindestens eine zweizähnige organische Verbindung mindestens ein Metallion durch Oxidation mindestens einer das entsprechende Metall enthaltenden Anode erzeugt wird.

Der Begriff "elektrochemische Herstellung" bezeichnet ein Herstellverfahren, bei dem die Bildung mindestens eines Reaktionsproduktes mit der Wanderung von elektrischen Ladungen oder dem Auftreten von elektrischen Potentialen verbunden ist.

Der Begriff "mindestens ein Metallion", wie er im Zusammenhang mit der elektrochemischen Herstellung verwendet wird, bezeichnet Ausführungsformen, gemäß denen mindestens ein Ion eines Metalls oder mindestens ein Ion eines ersten Metalls und mindestens ein Ion mindestens eines vom ersten Metall verschiedenen zweiten Metalls durch anodische Oxidation bereit gestellt werden.

Demgemäß umfasst die elektrochemische Herstellung Ausführungsformen, in denen mindestens ein Ion mindestens eines Metalls durch anodische Oxidation und mindestens ein Ion mindestens eines Metalls über ein Metallsalz bereit gestellt werden, wobei das mindestens eine Metall im Metallsalz und das mindestens eine Metall, das über anodische Oxidation als Metallion bereit gestellt werden, gleich oder voneinander verschieden sein können. Daher umfasst die vorliegende Erfindung in Bezug auf elektrochemisch hergestellte MOF beispielsweise eine Ausführungsform, gemäß der das Reaktionsmedium ein oder mehrere unterschiedliche Salze eines Metalls enthält und das in diesem Salz oder in diesen Salzen enthaltene Metallion zusätzlich durch anodische Oxidation mindestens einer dieses Metall enthaltenden Anode bereitgestellt wird. Ebenso kann das Reaktionsmedium ein oder mehrere unterschiedliche Salze mindestens eines Metalls enthalten und mindestens ein von diesen Metallen unterschiedliches Metall kann über anodische Oxidation als Metallion im Reaktionsmedium bereitgestellt werden.

Gemäß einer bevorzugten Ausführungsform der vorliegenden Erfindung im Zusammenhang mit der elektrochemischen Herstellung wird das mindestens eine Metallion durch anodische Oxidation mindestens einer der dieses mindestens eine Metall enthaltenden Anode bereitgestellt, wobei kein weiteres Metall über ein Metallsalz bereitgestellt wird.

Der Begriff "Metall", wie im Rahmen der vorliegenden Erfindung im Zusammenhang mit der elektrochemischen Herstellung von MOFs verwendet wird, umfasst sämtliche Elemente des Periodensystems, die über anodische Oxidation auf elektrochemischem Weg in einem Reaktionsmedium bereitgestellt werden können und mit mindestens einer mindestens zweizähnigen organischen Verbindungen mindestens ein metallorganisches poröses Gerüstmaterial zu bilden in der Lage sind.

Unabhängig von dessen Herstellung fällt das erhaltene MOF in pulverförmiger beziehungsweise kristalliner Form an. Dieses wird vorzugsweise als solches in der erfindungsgemäßen Suspension eingesetzt. Hierbei dient das metallorganische Gerüstmaterial als Sorbens. Weiterhin können in der Suspension auch andere Sorbentien eingesetzt werden. Prinzipiell kann das metallorganische Gerüstmaterial auch in einen Formkörper umgewandelt werden und dieser in der erfindungsgemäßen Suspension eingesetzt werden.

Ein weiterer Gegenstand der vorliegenden Erfindung ist die Verwendung der erfindungsgemäßen Suspension zur Vermindung von Geruch.

Nachfolgend wird die Erfindung durch das anschließende Beispiel näher erläutert.

### Beispiele

### Beispiel 1

Anhand eines Geruchstests wird die Wirkung einer erfindungsgemäßen Suspension mit verschiedenen metallorganischen Gerüstmaterialien im Hinblick auf die Verminderung von Geruch durch Geruchsstoffe aus Zigarettenrauch überprüft. Als Vergleich dienen eine Suspension aus β-Cyclodextrin sowie reines Wasser.

Es werden folgende Proben verwendet:
- Probe 1:: 0,5 Gew.-% β-Cyclodextrin in Wasser,
- Probe 2:: 0,5 Gew.-% MOF-5 in Wasser (Zn mit Terephthalsäure),
- Probe 3:: 0,5 Gew.-% IR-MOF-8 in Wasser (Zn mit 2,6-Naphthalindicarbonsäure),
- Probe 4:: 0,5 Gew.-% auf elektrochemischem Wege hergestelltes Kupfer-Gerüstmaterial gemäß Beispiel 2 von WO-A 2005/049892 sowie Wasser.

Als Apparatur dient ein 10 L Gefäß mit zwei verschließbaren, an entgegengesetzten Punkten befindlichen Öffnungen. Als Testgewebe wird Baumwollkörper Lg. Nr. 286 verwendet. Es wird weiterhin eine Zigarette der Marke Gautoises Blondes, blaue Packung (10 mg Teer, 0,8 mg Nikotin) verwendet. Zur Vorbereitung werden die Testgewebe in die Apparatur eingehängt. An der einen Öffnung wird eine Zigarette derart angebracht, dass Rauch durch den Filter der Zigarette in das Gefäß eingesaugt werden kann. An der anderen Öffnung wird ein Vakuum angelegt, um den Rauch in das Gefäß einsaugen zu können. Vakuum wird über ein T-Stück von Hand so eingeregelt, dass die Zigarette innerhalb von 2 Minuten möglichst gleichmäßig bis zum Anfang der Schrift abbrennt. Danach ist das Vakuum schnell von der Apparatur zu nehmen und die Apparatur ist beidseitig zu verschließen. Die Testgewebe werden nun 2 Stunden in der Apparatur belassen, auf 6 x 6 cm zugeschnitten und gegebenenfalls zur Lagerung in eine verschlossene Plastikflasche gegeben. Aus ca. 30 cm Entfernung werden die Testgewebe durch Aufsprühen der Proben 1 bis 4 sowie Wasser durch zwei Sprühstöße (ca. 2,5 ml) mit einem handelsüblichen Druckpumpzerstäuber derart in Kontakt gebracht, dass die gesamte Fläche des Testgewebes benetzt wird. Danach werden die Testgewebe 1 Stunde frei hängend bei Raumtemperatur getrocknet. Die Testgewebe werden hinsichtlich ihres Geruchs sofort in feuchtem Zustand (Tabelle A) und nach 6 Stunden in trockenem Zustand (Tabelle B) bewertet.

### Beurteilungsnote

| | |
|---|---|
| 1 = | ohne Geruch |
| 10 = | starker Zigarettengeruch |

**Tabelle A**

| Bewertende Person | Probe 1 | Probe 2 | Probe 3 | Probe 4 | Wasser |
|---|---|---|---|---|---|
| A | 4 | 8 | 5 | 5 | 10 |
| B | 3 | 10 | 6 | 7 | 10 |
| C | 5 | 7 | 4 | 6 | 10 |
| D | 4 | 7 | 7 | 4 | 9 |
| E | 4 | 8 | 4 | 4 | 10 |
| F | 5 | 6 | 4 | 4 | 10 |
| G | 3 | 9 | 5 | 5 | 10 |
| H | 4 | 8 | 5 | 4 | 10 |
| Durchschnitt | 4,0 | 7,9 | 5 | 4,9 | 9,9 |

**Tabelle B**

| Bewertende Person | Probe 1 | Probe 2 | Probe 3 | Probe 4 | Wasser |
|---|---|---|---|---|---|
| A | 6 | 8 | 3 | 4 | 10 |
| B | 5 | 7 | 4 | 5 | 10 |
| C | 10 | 4 | 5 | 5 | 10 |
| D | 5 | 5 | 3 | 3 | 9 |
| E | 6 | 8 | 3 | 2 | 10 |
| F | 6 | 9 | 1 | 3 | 10 |
| G | 5 | 10 | 2 | 1 | 10 |
| H | 5 | 7 | 1 | 2 | 10 |
| Durchschnitt | 6,0 | 7,4 | 2,8 | 3,5 | 9,9 |

### Beispiel 2

- Getestete Proben:: Wasser, Proben 1 bis 3 gemäß Beispiel 1
- Reagenzien :: Baumwoll Köper Lg. Nr. 286 Zigarette (Gauloises Blondes, blaue Packung, 10mg Teer, 0,8 mg Nikotin,.)
- Vorbereitung:: Die Testgewebe werden in die in Beispiel 1 beschriebene Apparatur eingehängt. An der einen Öffnung wird eine Zigarette derart angebracht, dass Rauch durch den Filter der Zigarette in das Gefäß eingesaugt werden kann. An der anderen Öffnung wird ein Vakuum angelegt um den Rauch in das Gefäß einsaugen zu können. Vakuum wird über ein T-Stück von Hand so eingeregelt, das die Zigarette innerhalb 2 Minuten möglichst gleichmäßig bis zum Anfang der Schrift abbrennt. Danach ist das Vakuum möglichst schnell von der Apparatur zu nehmen und die Apparatur ist beidseitig zu verschließen. Die Testgewebe werden nun zwei Stunden in der Apparatur belassen, auf 6x6 cm zugeschnitten, und ggf. zur Lagerung in eine verschlossene Plastikflasche gegeben.
- Durchführung:: Die Testgewebe werden auf einem Filterpapier befestigt, welches vertikal an eine nicht saugfähige Wand aufgehängt wird. Aus 30 cm Entfernung werden je Gewebe und zu prüfender Probe 2 Sprühstöße (ca. 2,5 ml). mit dem Zerstäuber derart aufgetragen, dass die gesamte Fläche des Testgewebes benetzt wird. Danach werden die Testgewebe 30 min. freihängend bei Raumtemperatur getrocknet. Die Testgewebe werden hinsichtlich ihres Geruches bewertet. Das Ergebnis ist in der nachfolgenden Tabelle wiedergegeben.
Beurteilungsnote 1 = ohne Geruch 10 = starker Zigarettengeruch

| **Bewertende Person** | **Raucher** | **Unbehandelt** | **Wasser** | **Probe 1** | **Probe 2** | **Probe 3** |
|---|---|---|---|---|---|---|
| A | Nein | 7 | 7 | 8 | 6 | 5 |
| B | Ja | 7 | 8 | 6 | 5 | 5 |
| C | Nein | 7 | 8 | 7 | 6 | 6 |
| D | Nein | 9 | 8 | 6 | 8 | 4 |
| E | Nein | 9 | 8 | 8 | 8 | 7 |
| F | Nein | 8 | 7 | 5 | 7 | 5 |
| G | Ja | 9 | 6 | 9 | 3 | 8 |
| H | Ja | 8 | 6 | 5 | 5 | 7 |
| I | Ja | 9 | 7 | 4 | 5 | 6 |
| J | Nein | 10 | 6 | 3 | 2 | 2 |
| | | | | | | |
| **Durchschnitt** | | 9,3 | 8,1 | 7,1 | 6,6 | 6,7 |

## Patentansprüche

1. Suspension zur Verminderung von Geruch enthaltend ein poröses metallorganisches Gerüstmaterial in einer Flüssigkeit, wobei das Gerüstmaterial mindestens eine an mindestens ein Metallion koordinativ gebundene, mindestens zweizähnige organische Verbindung enthält, wobei die Konzentration des Gerüstmaterials im Bereich von 0,001 bis 5 Gew.-% bezogen auf das Gesamtgewicht der Suspension liegt und wobei die Flüssigkeit Wasser enthält oder Wasser ist.

2. Suspension nach Anspruch 1, **dadurch gekennzeichnet, dass** das Gerüstmaterial Zn, Al, Ni oder Cu als Metallion enthält und die mindestens zweizähnige organische Verbindung Terephthalsäure, Isophthalsäure, 2,6-Naphthalindicarbonsäure oder 1,3,5-Benzoltricarbonsäure ist.

3. Zerstäuber enthaltend eine Suspension nach Anspruch 1 oder 2.

4. Verfahren zur Verminderung von Geruch, den Schritt enthaltend
- Inkontaktbringen eines den Geruch enthaltenden Gases oder an der Oberfläche eines Gegenstandes oder an einem Lebewesen haftenden Geruches mit einer Suspension nach Anspruch 1 oder 2.

5. Verfahren nach Anspruch 4, **dadurch gekennzeichnet, dass** der Geruch Küchengeruch, Schweißgeruch, Inkontinenzgeruch, Lebensmittelgeruch oder Toilettengeruch ist oder durch Tabakrauch verursacht wird.

6. Verfahren nach Anspruch 4 oder 5, **dadurch gekennzeichnet, dass** der Geruch durch mindestens einen Geruchsstoff verursacht wird, wobei der Geruchsstoff eine flüchtige organische oder anorganische Verbindung, die mindestens eines der Elemente Stickstoff, Phosphat, Sauerstoff, Schwefel, Fluor, Chlor, Brom oder Iod enthält oder ein ungesättigter oder aromatischer Kohlenwasserstoff oder ein gesättigter oder ungesättigter Aldehyd oder Keton ist.

7. Verfahren nach einem der Ansprüche 4 bis 6, **dadurch gekennzeichnet, dass** das Gas Luft ist.

8. Verwendung einer Suspension nach Anspruch 1 oder 2 zur Verminderung von Geruch.

## Claims

1. A suspension for odor reduction comprising a porous metal-organic framework material in a liquid, the framework material comprising at least one, at least bidentate, organic compound bound by coordination to at least one metal ion, the concentration of the framework material being in the range from 0.001 to 5% by weight based on the total weight of the suspension and the liquid comprising water or being water.

2. The suspension according to claim 1, wherein the framework material comprises Zn, Al, Ni or Cu as metal ion and the at least bidentate organic compound is terephthalic acid, isophthalic acid, 2,6-naphthalenedicarboxylic acid or 1,3,5-benzenetricarboxylic acid.

3. An atomizer comprising a suspension according to claim 1 or 2.

4. A method for odor reduction comprising the step of
- contacting a gas comprising the odor, or an odor adhering to the surface of an article or to an organism with a suspension according to claim 1 or 2.

5. The method according to claim 4, wherein the odor is kitchen odor, sweat odor, incontinence odor, food odor or toilet odor, or is caused by tobacco smoke.

6. The method according to claim 4 or 5, wherein the odor is caused by at least one odor substance, the odor substance being a volatile organic or inorganic compound which comprises at least one of the elements nitrogen, phosphate, oxygen, sulfur, fluorine, chlorine, bromine or iodine, or is an unsaturated or aromatic hydrocarbon, or a saturated or unsaturated aldehyde or ketone.

7. The method according to any of claims 4 to 6, wherein the gas is air.

8. The use of a suspension according to claim 1 or 2 for odor reduction.

## Revendications

1. Suspension pour l'atténuation d'une odeur contenant un matériau de squelette métallo-organique poreux dans un liquide, le matériau de squelette contenant au moins un composé organique au moins bidentate, relié coordinativement à au moins un ion métallique, la concentration du matériau de squelette étant dans la plage allant de 0,001 à 5 % en poids par rapport au poids total de la suspension et le liquide contenant de l'eau ou étant de l'eau.

2. Suspension selon la revendication 1, **caractérisée en ce que** le matériau de squelette contient Zn, Al, Ni ou Cu en tant qu'ion métallique et le composé organique au moins bidentate est l'acide téréphtalique, l'acide isophtalique, l'acide 2,6-naphtalinedicarboxylique ou l'acide 1,3,5-benzènetricarboxylique.

3. Brumisateur contenant une suspension selon la revendication 1 ou 2.

4. Procédé d'atténuation d'une odeur, contenant l'étape
- mise en contact d'un gaz contenant l'odeur ou d'une odeur adhérente à la surface d'un objet ou à un être vivant avec une suspension selon la revendication 1 ou 2.

5. Procédé selon la revendication 4, **caractérisé en ce que** l'odeur est une odeur de cuisine, une odeur de transpiration, une odeur d'incontinence, une odeur de produit alimentaire ou une odeur de toilettes ou est causée par la fumée de tabac.

6. Procédé selon la revendication 4 ou 5, **caractérisé en ce que** l'odeur est causée par au moins une substance odorante, la substance odorante étant un composé volatil organique ou inorganique, qui contient au moins un des éléments azote, phosphate, oxygène, soufre, fluor, chlore, brome ou iode, ou un hydrocarbure insaturé ou aromatique, ou une cétone ou un aldéhyde saturé ou insaturé.

7. Procédé selon l'une quelconque des revendications 4 à 6, **caractérisé en ce que** le gaz est l'air.

8. Utilisation d'une suspension selon la revendication 1 ou 2 pour l'atténuation d'une odeur.
